(19) Europäisches Patentamt
European Patent Office
Office européen des brevets

(11) EP 4 368 647 A1

(12) EUROPEAN PATENT APPLICATION
published in accordance with Art. 153(4) EPC

(43) Date of publication:
**15.05.2024 Bulletin 2024/20**

(21) Application number: **22837604.2**

(22) Date of filing: **30.06.2022**

(51) International Patent Classification (IPC):
***C08F 216/14*** *(2006.01)* ***C07C 309/82*** *(2006.01)*
***C08F 214/18*** *(2006.01)*

(52) Cooperative Patent Classification (CPC):
**C07C 309/82; C08F 214/18; C08F 216/14;**
Y02E 60/50

(86) International application number:
**PCT/JP2022/026340**

(87) International publication number:
**WO 2023/282188 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2021 JP 2021112875**

(71) Applicant: **AGC INC.**
**Chiyoda-ku,**
**Tokyo 1008405 (JP)**

(72) Inventors:
- **SHIONO Takeshi**
  **Tokyo 100-8405 (JP)**
- **HIRAI Takeshi**
  **Tokyo 100-8405 (JP)**

(74) Representative: **Müller-Boré & Partner Patentanwälte PartG mbB**
**Friedenheimer Brücke 21**
**80639 München (DE)**

# (54) PERFLUORO COMPOUND PRODUCTION METHOD AND FLUORINE-CONTAINING POLYMER PRODUCTION METHOD

(57) To provide a method for producing a perfluorinated compound, capable of easily producing a perfluorinated compound from which a high molecular weight polymer can be produced by a polymerization reaction, and a method for producing a fluorinated polymer using it.

A method for producing a perfluorinated compound, which comprises step A of reacting a perfluoroalkoxide salt with a perfluoroallylating agent to obtain a crude product A containing a perfluoroallyl ether compound, or step B of subjecting a chlorofluorocarbon compound to dechlorination reaction to obtain a crude product B containing a perfluorinated α-olefin compound, to obtain a crude product X which is either the crude product A or the crude product B, and
mixing the crude product X with a radical source to bring radicals generated by the radical source and the crude product X into contact with each other to obtain a crude product Y containing a perfluorinated compound which is either the perfluoroallyl ether compound or the perfluorinated α-olefin compound.

EP 4 368 647 A1

## Description

TECHNICAL FIELD

The present invention relates to a method for producing a perfluorinated compound, and a method for producing a fluorinated polymer.

BACKGROUND ART

Perfluorinated compounds such as a perfluoroallyl ether compound and a perfluorinated α-olefin compound are used, for example, as a monomer for producing a fluorinated polymer.

As a method for producing a fluorinated polymer, Patent Document 1 discloses a method of copolymerizing tetrafluoroethylene and a monomer represented by $CF_2=CFCF_2OCF_2CF_2SO_2F$ in the presence of a radical polymerization initiator at a temperature of 100 to 200°C, and hydrolyzing the $-SO_2F$ group into a sulfonic acid group to convert the polymer into an acid form.

PRIOR ART DOCUMENTS

PATENT DOCUMENTS

Patent Document 1: JP-A-2010-18674

DISCLOSURE OF INVENTION

TECHNICAL PROBLEM

A polymer obtained from a perfluorinated compound may sometimes be required to have a high molecular weight depending upon its application. For example, in a case where the polymer is used for production of an electrolyte membrane, if the polymer has a low molecular weight, the electrolyte membrane tends to have insufficient mechanical strength. Thus, particularly in such a field, a high molecular weight polymer is required.

The present inventors have produced a polymer with reference to the production method described in Patent Document 1 and found that the molecular weight is insufficient and has to be improved.

The present invention has been made to overcome the above problem, and its object is to provide a method for producing a perfluorinated compound, capable of easily producing a perfluorinated compound from which a high molecular weight polymer can be produced by polymerization reaction, and a method for producing a fluorinated polymer using it.

SOLUTION TO PROBLEM

The present inventors have conducted extensive studies to achieve the above object and as a result found that by a method for producing a perfluorinated compound, which comprises obtaining a crude product X containing a perfluorinated compound which is either a perfluoroallyl ether compound or a perfluorinated α-olefin compound by a synthesis reaction, and mixing the crude product X with a radical source to bring radicals generated by the radical source and the crude product X into contact with each other, a perfluorinated compound from which a high molecular polymer can be produced by a polymerization reaction using the perfluorinated compound, and accomplished the present invention.

That is, the present inventors have found that the above object can be achieved by the following constitution.

[1] A method for producing a perfluorinated compound, which comprises step A of reacting a perfluoroalkoxide salt with a perfluoroallylating agent to obtain a crude product A containing a perfluoroallyl ether compound, or step B of subjecting a chlorofluorocarbon compound to dechlorination reaction to obtain a crude product B containing a perfluorinated α-olefin compound, to obtain a crude product X which is either the crude product A or the crude product B, and
mixing the crude product X with a radical source to bring radicals generated by the radical source and the crude product X into contact with each other to obtain a crude product Y containing a perfluorinated compound which is either the perfluoroallyl ether compound or the perfluorinated α-olefin compound.

[2] The method for producing a perfluorinated compound according to [1], wherein mixing of the crude product X with the radical source is conducted under conditions where substantially no polymerizable compound other than

the polymerizable compound contained in the crude product X is present.
[3] The method for producing a perfluorinated compound according to [1] or [2], wherein the denaturation ratio D calculated by the following formula (M) is 15% or less:

$$\text{denaturation ratio D [\%]}=100\times(X-Y)/X \quad (M)$$

wherein X is the proportion (%) of the peak area of the perfluorinated compound to the total area of all peaks detected in gas chromatography of the crude product X, and Y is the proportion (%) of the peak area of the perfluorinated compound to the total area of all peaks detected in gas chromatography of the crude product Y.
[4] The method for producing a perfluorinated compound according to any one of [1] to [3], wherein the perfluoroalkoxide salt is a compound represented by the following formula (S1), and the perfluoroallylating agent is a compound represented by the following formula (S2):

$CF(-Q^{11})(-Q^{12})-O-M$ (S1)

$CF_2=CFCF_2-Z^S$ (S2)

in the formula (S1), $Q^{11}$ and $Q^{12}$ are each independently a perfluoroalkyl group which may have $-SO_2F$, a monovalent group having $-CF_2-$ in a perfluoroalkyl group which may have $-SO_2F$ replaced with an etheric oxygen atom, or a fluorine atom, and M is a monovalent metal element;
in the formula (S2), $Z^S$ is $-OS(O)_2R^{S1}$, a chlorine atom, a bromine atom or an iodine atom, and $R^{S1}$ is a fluorine atom or a perfluoroalkyl group.

[5] The method for producing a perfluorinated compound according to [4], wherein the perfluoroallyl ether compound obtained by reacting the compound represented by the formula (S1) with the compound represented by the formula (S2) is a compound represented by the following formula (F1):

$CF(-Q^{11})(-Q^{12})-O-CF_2CF=CF_2$ (F1)

in the formula (F1), definitions of $Q^{11}$ and $Q^{12}$ are respectively the same as those of $Q^{11}$ and $Q^{12}$ in the formula (S1).
[6] The method for producing a perfluorinated compound according to [4], wherein the compound represented by the formula (S1) is a compound represented by the following formula (S1-1) or a compound represented by the following formula (S1 -2):

$FO_2S-Q^{21}-CF_2-O-M$ (S1-1)

$CF(-Q^{22}-SO_2F)(-Q^{23}-SO_2F)-O-M$ (S1-2)

in the formula (S1-1), $Q^{21}$ is a perfluoroalkylene group, or a bivalent group having - $CF_2$- in a perfluoroalkylene group replaced with an etheric oxygen atom, and
in the formula (S1-2), $Q^{22}$ and $Q^{23}$ are each independently a perfluoroalkylene group, or a bivalent group having $-CF_2-$ in a perfluoroalkylene group replaced with an etheric oxygen atom.

[7] The method for producing a perfluorinated compound according to [6], wherein the perfluoroallyl ether compound obtained by reacting the compound represented by the formula (S1-1) with the compound represented by the formula (S2) is a compound represented by the following formula (F1-1), and the perfluoroallyl ether compound obtained by reacting the compound represented by the formula (S1-2) and the compound represented by the formula (S2) is a compound represented by the following formula (F1-2).

$FO_2S-Q^{21}-CF_2-O-CF_2CF=CF_2$ (F1-1)

$CF(-Q^{22}-SO_2F)(-Q^{23}-SO_2F)-O- CF_2CF=CF_2$ (F1-2)

in the formula (F1-1), definition of $Q^{21}$ is the same as that of $Q^{21}$ in the formula (S1-1); and

in the formula (F1-2), definitions of $Q^{22}$ and $Q^{23}$ are respectively the same as those of $Q^{22}$ and $Q^{23}$ in the formula (S1-2).

[8] The method for producing a perfluorinated compound according to any one of [1] to [3], wherein the chlorofluorocarbon compound is a compound represented by the following formula (S3):

$ClCF_2$-CFCl-$CF_2$-$CF_2$-$Q^{13}$-$SO_2F$ (S3)

in the formula (S3), $Q^{13}$ is a single bond, a perfluoroalkylene group which may have -$SO_2F$, or a bivalent group having -$CF_2$- in a perfluoroalkylene group which may have -$SO_2F$ replaced with an etheric oxygen atom.
[9] The method for producing a perfluorinated compound according to [8], wherein the perfluorinated $\alpha$-olefin compound obtained by subjecting the chlorofluorocarbon compound to dechlorination reaction is a compound represented by the following formula (F2).

$CF_2$=CF-$CF_2$-$CF_2$-$Q^{13}$-$SO_2F$ (F2)

in the formula (F2), definition of $Q^{13}$ is the same as that of $Q^{13}$ in the formula (S3).
[10] The method for producing a perfluorinated compound according to [9], wherein the compound represented by the formula (F2) is a compound represented by the following formula (F2-1).

$CF_2$=CF-$(CF_2CF_2)_{X2}$-$SO_2F$ (F2-1)

in the formula (F2-1), x2 is an integer of from 1 to 6.
[11] The method for producing a perfluorinated compound according to any one of [1] to [10], wherein the crude product Y is further purified.
[12] The method for producing a perfluorinated compound according to [11], wherein mixing of the crude product X with the radical source, and purification of the crude product Y, are conducted in the same apparatus.
[13] A method for producing a fluorinated polymer, which comprises polymerizing the perfluorinated compound obtained by the method for producing a perfluorinated compound as defined in any one of [1] to [12] at 100°C or higher to obtain a fluorinated polymer.

ADVANTAGEOUS EFFECTS OF INVENTION

According to the present invention, it is possible to provide a method for producing a perfluorinated compound, capable of easily producing a perfluorinated compound from which a high molecular weight polymer can be produced by a polymerization reaction, and a method for producing a fluorinated polymer using it.

DESCRIPTION OF EMBODIMENTS

Meanings of terms in this specification are as follows.
An $\alpha$-olefin means an olefin having a carbon-carbon double bond at the terminal ($\alpha$) carbon.

[Method for producing perfluorinated compound]

The method for producing a perfluorinated compound of the present invention has step A of reacting a perfluoroalkoxide salt with a perfluoroallylating agent to obtain a crude product A containing a perfluoroallyl ether compound, or step B of subjecting a chlorofluorocarbon compound to a dechlorination reaction to obtain a crude product B containing a perfluorinated $\alpha$-olefin compound. By such a step, a crude product X, which is either the crude product A or the crude product B is obtained.
The method for producing a perfluorinated compound of the present invention has a step of mixing the crude product X with a radical source to bring radicals generated by the radical source and the crude product X into contact with each other to obtain a crude product Y containing a perfluorinated compound which is either the perfluoroallyl ether compound or the perfluorinated $\alpha$-olefin compound (hereinafter sometimes referred to as "step C"). In the following, the step C conducted by using the crude product A may sometimes be referred to as "step C1", and the obtainable product may sometimes be referred to as "crude product C1". Further, the step C conducted by using the crude product B may sometimes be referred to as "step C2", and the obtainable product may sometimes be referred to as "crude product C2". The crude product Y is either the crude product C1 or the crude product C2.
According to the present production method, a perfluorinated compound from which a high molecular weight

polymer can be produced by a polymerization reaction, can easily be obtained. "A perfluorinated compound can easily obtained" means that the perfluorinated compound can be produced with an excellent production efficiency at a low cost.

The reason has not been clearly understood in detail yet, however, it is estimated generally as follows.

In a reaction for synthesis of the perfluoroallyl ether compound and the perfluorinated $\alpha$-olefin compound, various compounds are synthesized by side reactions in the process of the synthesis reaction and are included as impurities in the crude product X. When a solvent is used for the reaction, the remaining solvent and decomposed products of the solvent are also included as impurities in the crude product X. Such impurities included in the crude product X may undergo chain transfer or the like when the crude product X is used for polymerization, and thus a high molecular weight polymer may sometimes be difficult to obtain. This problem tends to be remarkable particularly when the polymerization temperature is high, and may be a serious problem.

To solve this problem, separation of the impurities from the perfluorinated compound may be mentioned, however, the impurities may sometimes have a degree of volatility close to that of the perfluorinated compound. Accordingly, in order to obtain a perfluorinated compound from which a high molecular weight polymer can be obtained by polymerization, by separation from the impurities included in the crude product X, there are problems in view of purification efficiency and cost, such that use of a distillation column with a high number of theoretical plate is required.

To overcome such a problem, the present inventors have estimated that by the above step C, the impurities in the crude product X are decomposed or denatured by the action of radicals and as a result, a perfluorinated compound from which a high molecular weight polymer can be produced by a polymerization reaction, is obtained.

In the following, an embodiment in which the method for producing a perfluorinated compound of the present invention has the step A and the step C1 is taken as a first embodiment, and an embodiment in which the method for producing a perfluorinated compound of the present invention has the step B and the step C2 is taken as a second embodiment, and the respective embodiments will be described below.

[First embodiment]

The method for producing a perfluorinated compound according to the first embodiment has the step A and the step C1.

<Step A>

The step A is a step of reacting a perfluoroalkoxide salt with a perfluoroallylating agent to obtain a crude product A containing a perfluoroallyl ether compound.

The perfluoroalkoxide salt is preferably a compound represented by the following formula (S1) (hereinafter sometimes referred to as "compound (S1)").

$$CF(-Q^{11})(-Q^{12})\text{-}O\text{-}M \qquad (S1)$$

In the formula (S1), $Q^{11}$ and $Q^{12}$ are each independently a perfluoroalkyl group which may have $-SO_2F$, a monovalent group having $-CF_2-$ in a perfluoroalkyl group which may have $-SO_2F$ replaced with an etheric oxygen atom, or a fluorine atom.

The perfluoroalkyl group may be linear or branched.

The number of carbon atoms in the perfluoroalkyl group is, with a view to suppressing a decrease of the ion exchange capacity of the polymer, preferably from 1 to 12, more preferably from 1 to 6, particularly preferably from 1 to 4.

In a case where the perfluoroalkyl group has $-SO_2F$, the number of $-SO_2F$ may be one or more. $-SO_2F$ may be bonded to any carbon atom, however, it is preferably bonded to the terminal carbon atom.

The number of the etheric oxygen atom in the monovalent group may be one or more, and is preferably two or less. The etheric oxygen atom is preferably located in a carbon-carbon bond in the perfluoroalkyl group.

In the formula (S1), M is a monovalent metal element. The monovalent metal element may be Na, K, Cs or Ag and is preferably K.

The compound (S1) preferably has $-SO_2F$ in that a perfluoroallyl ether compound suitable for production of a polymer for an electrolyte membrane is obtained, and is particularly preferably a compound represented by the following formula (S1-1) (hereinafter sometimes referred to as "compound (S1-1)"), or a compound represented by the following formula (S1-2) (hereinafter sometimes referred to as "compound (S1-2)").

$$FO_2S\text{-}Q^{21}\text{-}CF_2\text{-}O\text{-}M \qquad (S1\text{-}1)$$

$$CF(-Q^{22}\text{-}SO_2F)(-Q^{23}\text{-}SO_2F)\text{-}O\text{-}M \qquad (S1\text{-}2)$$

In the formula (S1-1), $Q^{21}$ is a perfluoroalkylene group, or a bivalent group having $-CF_2-$ in a perfluoroalkylene group replaced with an etheric oxygen atom.

The perfluoroalkylene group may be linear or branched.

The number of carbon atoms in the perfluoroalkylene group is, with a view to suppressing a decrease of the ion exchange capacity of the polymer, preferably from 1 to 12, more preferably from 1 to 6, particularly preferably from 1 to 4.

The number of the etheric oxygen atom in the bivalent group may be one or more, and is preferably two or less. The etheric oxygen atom is preferably located in a caron-carbon bond of the perfluoroalkylene group. In the formula (S1 -1), $Q^{21}$ is most preferably $CF_2$.

In the formula (S1-2), $Q^{22}$ and $Q^{23}$ are each independently a perfluoroalkylene group, or a bivalent group having $-CF_2-$ in a perfluoroalkylene group replaced with an etheric oxygen atom.

The perfluoroalkylene group may be linear or branched.

The number of carbon atoms in the perfluoroalkylene group is, with a view to suppressing a decrease of the ion exchange capacity of the polymer, preferably from 1 to 12, more preferably from 1 to 6, particularly preferably from 1 to 4.

The number of the etheric oxygen atom in the bivalent group may be one or more, and is preferably two or less. The etheric oxygen atom is preferably located in a carbon-carbon bond of the perfluoroalkylene group. In the formula (S1-2), $Q^{22}$ and $Q^{23}$ are respectively most preferably $CF_2$.

The perfluoroallylating agent is preferably a compound represented by the following formula (S2) (hereinafter sometimes referred to as "compound (S2)").

$$CF_2=CFCF_2-Z^S \qquad (S2)$$

In the formula (S2), $Z^S$ is $-OS(O)_2R^{S1}$, a chlorine atom, a bromine atom or an iodine atom. $R^{S1}$ is a fluorine atom or a perfluoroalkyl group.

The perfluoroalkyl group may be linear or branched.

The number of carbon atoms in the perfluoroalkyl group is preferably from 1 to 4, more preferably from 1 to 2, particularly preferably 1.

The step A is carried out preferably in the presence of an organic solvent. Specific examples of the organic solvent include ethylene glycol dimethyl ether (monoglyme), diethylene glycol dimethyl ether (diglyme), triethylene glycol dimethyl ether (triglyme), tetraethylene glycol dimethyl ether (tetraglyme) or acetonitrile.

In the step A, a fluoride such as potassium fluoride may further be used.

The reaction conditions in the step A are not particularly limited, and known reaction conditions may be employed.

(Crude product A)

The crude product A obtained by the step A contains the perfluoroallyl ether compound which is a desired product and is one type of the perfluorinated compound, and further includes impurities other than the perfluorinated compound.

The impurities in the crude product A may, for example, be various compounds formed by side reactions and the organic solvent used in the step A.

The amount of the perfluoroallyl ether compound contained in the crude product A is, to the total mass of the crude product A, preferably 30 mass% or more, more preferably 50 mass% or more, further preferably 85 mass% or more, particularly preferably 95 mass% or more.

The amount of the perfluoroallyl ether compound contained in the crude product A is preferably large in view of the production efficiency, however, even if it is small, a perfluoroallyl ether compound with a higher purity can be obtained e.g. by the after-described purification. Thus, a case where the amount of the perfluoroallyl ether compound contained in the crude product A is small is also acceptable.

(Perfluoroallyl ether compound)

The perfluoroallyl ether compound is preferably a compound represented by the following formula (F1) (hereinafter sometimes referred to as "monomer (F1)").

$$CF(-Q^{11})(-Q^{12})-O-CF_2CF=CF_2 \qquad (F1)$$

In the formula (F1), definitions of $Q^{11}$ and $Q^{12}$ are respectively the same as those of $Q^{11}$ and $Q^{12}$ in the formula (S1).

The monomer (F1) preferably has $-SO_2F$, whereby it is suitable for production of a polymer for an electrolyte membrane, and is preferably a compound represented by the following formula (F1-1) (hereinafter sometimes referred to as "monomer (F1-1)"), or a compound represented by the following formula (F1-2) (hereinafter sometimes referred to as "monomer (F1-2)").

$$FO_2S\text{-}Q^{21}\text{-}CF_2\text{-}O\text{-}CF_2CF{=}CF_2 \qquad \text{(F1-1)}$$

$$CF(\text{-}Q^{22}\text{-}SO_2F)(\text{-}Q^{23}\text{-}SO_2F)\text{-}O\text{-}CF_2CF{=}CF_2 \qquad \text{(F1-2)}$$

In the formula (F1-1), definition of $Q^{21}$ is the same as that of $Q^{21}$ in the formula (S1-1).
In the formula (F1-2), definitions of $Q^{22}$ and $Q^{23}$ are respectively the same as those of $Q^{22}$ and $Q^{23}$ in the formula (S1-2).

<Step C1>

The step C1 is a step of mixing the crude product A with a radical source to bring radicals generated by the radical source and the crude product A into contact with each other to obtain a crude product C1 containing the perfluoroallyl ether compound.
The crude product C1 obtained by the step C1 is considered to include, in addition to the desired product perfluoroallyl ether compound, impurities originally included in the crude product A and impurities generated by decomposition or denaturation of the originally included impurities by the action of radicals. It is estimated that a higher molecular weight polymer is obtained by polymerizing the perfluoroallyl ether compound since the impurities included in the crude product A become inactive to radicals by decomposition or denaturation by the action of radicals.
The radical source may be an organic peroxide, a nitrogen-containing compound, a persulfate, a halogenated compound, a sulfur-containing compound, a nitrogen-sulfur bond-containing compound, hydrogen peroxide, iodine, chlorine, bromine or sulfur.
The organic peroxide may, for example, be a diacyl peroxide, an alkyl peroxyester, a peroxydicarbonate, a peroxycarbonate, a peroxyketal, a dialkyl peroxide or a hydroperoxide. The nitrogen-containing compound may, for example, be an azo compound or an amine compound. The sulfur-containing compound may, for example, be a disulfide, a monosulfide, a sulfinic acid or a dialkylsulfone. The nitrogen-sulfur bond-containing compound may, for example, be a sulfenamide.
Among them, in order that a radical source which is the same as an initiator used for polymerization can be used, the radical source is preferably an organic peroxide, more preferably a dialkyl peroxide, particularly preferably a bis(perfluoroalkyl) peroxide (such as $(CF_3)_3COOC(CF_3)_3$) or a dialkyl peroxide (such as $(CH_3)_3COOC(CH_3)_3$).
The radical source may be used in combination of two or more.
The amount of the radical source added is, in view of more excellent effects of the present invention, preferably from 0.01 to 2 parts by mass, more preferably from 0.01 to 1 parts by mass, particularly preferably from 0.03 to 0.3 parts by mass, per 100 parts by mass of the crude product A.
The step C1 is conducted preferably under heating conditions.
The heating temperature is preferably from 120 to 180°C, particularly preferably from 150 to 170°C. When the heating temperature is the lower limit value or more, the heating time can be shortened, and production of the perfluorinated compound can be conducted efficiently. Further, when the heating temperature is the upper limit value or lower, decomposition or denaturation of the perfluorinated compound itself can be suppressed.
The heating time is preferably from 1 to 120 hours, particularly preferably from 1 to 10 hours. When the heating time is the lower limit value or more, remining of the radical source can be suppressed. Further, when the heating time is the upper limit value or less, production of the perfluorinated compound can be conducted efficiently.
Mixing of the crude product A with the radical source is conducted preferably under conditions where substantially no polymerizable compound other than the polymerizable compound contained in the crude product A (hereinafter sometimes referred to as "other polymerizable compound A") is present, whereby consumption of radicals by other polymerizable compound A can be suppressed, and the reaction of radicals with impurities in the crude product A will be conducted more favorably.
The polymerizable compound here means a compound having a polymerizable group. The polymerizable group may, for example, be a vinyl group or a fluorovinyl group.
The polymerizable compound contained in the crude product A may, for example, be the perfluoroallyl ether compound, and a compound having a polymerizable group in the impurities. The compound having a polymerizable group in the impurities may, for example, be by-products formed by the step A.
The other polymerizable compound A may, for example, be tetrafluoroethylene, chlorotrifluoroethylene, trifluoroethylene, vinylidene fluoride, vinyl fluoride, ethylene or propylene.

In the step C1 "conditions where substantially no other polymerizable compound A is present", means that the content of the other polymerizable compound A is 0.1 parts by mass or less per 100 parts by mass of the crude product A used in the step C1.

The amount of the perfluoroallyl ether compound contained in the crude product C1 is, to the total mass of the crude product C1, preferably 30 mass% or more, more preferably 50 mass% or more, further preferably 85 mass% or more, particularly preferably 95 mass% or more.

The amount of the perfluoroallyl ether compound contained in the crude product C1 is preferably large in view of production efficiency, however, even if it is small, a perfluoroallyl ether compound with a higher purity can be obtained e.g. by the after-described purification. Thus, a case where the amount of the perfluoroallyl ether compound contained in the crude product C1 is small is also acceptable.

In order to decompose or denature the impurities by the action of radicals as described above, the contact of radicals and the crude product A in the step C1 has to be conducted at an appropriate temperature for an adequate time, and at the time of the contact, by the action of the radicals, the perfluoroallyl ether compound contained in the crude product A may be decomposed. Decomposition of the perfluoroallyl ether compound is unfavorable in view of efficiency, however, under conditions where decomposition of the perfluoroallyl ether compound is suppressed, decomposition or denaturation of the impurities may sometimes be insufficient.

Accordingly, in the present invention, in order to sufficiently decompose or denature the impurities, it is preferred to conduct the step C1 under conditions where the perfluoroallyl ether compound is decomposed to a certain extent. Specifically, the contact conditions are preferably such that the denaturation ratio D is a certain value or less.

The denaturation ratio D calculated by the following formula (M) is preferably as low as possible, whereby the yield of the perfluorinated compound increases. Specifically, the denaturation ratio D is preferably 20 % or less, more preferably 15 % or less, particularly preferably 5 % or less.

The lower limit of the denaturation ratio D is preferably 0%, however, in order that the impurities are securely decomposed or denatured, it is preferably 0.5%, more preferably 1.0%.

$$\text{Denaturation ratio D [\%]}=100\times(X-Y)/X \quad (M)$$

In the formula (M), X is the proportion (%) of the peak area of the perfluoroallyl ether compound, to the total area of all peaks detected, in gas chromatography of the crude product A obtained in the step A.

In the formula (M), Y is the proportion (%) of the peak area of the perfluoroalkyl ether compound, to the total area of all peaks detected, in gas chromatography of the crude product C1 obtained in the step C1.

The denaturation ratio D can easily be adjusted to be the above value by adjusting the amount of the radical source added, the temperature and the time.

The crude product C1 containing the perfluoroallyl ether compound thus obtained is preferably subjected to the after-described step D1, or it may be used as it is for the polymerization reaction. In such a case, it is preferred that the influence of the impurities included in the crude product C1 over the polymerization is small, and for example, it is preferred to use a perfluoroperoxide as the radical source, or to use the same solvent for the solvent used in the step A and the solvent for polymerization of the perfluoroallyl ether compound.

<Step D1>

The method for producing a perfluorinated compound according to the first embodiment preferably has, after the step C1 in which the crude product C1 is obtained, step D1 to purify the crude product C1, whereby a perfluorinated compound, the perfluoroallyl ether compound in the first embodiment, which has a high purity and is thereby used suitably for polymerization, can be obtained.

The purification method is not particularly limited and may, for example, be washing with water, distillation, extraction with a solvent or adsorption of impurities by a desiccant or an adsorbent, or a combination thereof. Among them, distillation is preferred, whereby a high purity perfluorinated compound can be easily recovered.

Distillation is conducted preferably by using a packed column or a distillation apparatus having a plate column. The number of theoretical plate of the packed column or the plate column is preferably from 10 to 100, particularly preferably from 10 to 80. According to the method for producing a perfluorinated compound in the first embodiment, which has the step C1, a high purity perfluorinated compound can be obtained even when the number of theoretical plate of the packed column or the plate column is small. The packing in the packed column may be structured packing or random packing. The packing may, for example, be HELIPACK, COIL PACK, PALL Ring, DIXON Packing or GOODROLL Packing (each manufactured by TO-TOKU ENGINEERING CO., LTD.)

The amount of the perfluoroallyl ether compound contained in the purified product obtained by the step D1 is, to the total mass of the purified product, preferably from 95 to 100 mass%, particularly preferably from 99 to 100 mass%.

It is preferred that the step C1 and the step D1 are conducted in the same apparatus, whereby production of the perfluorinated compound will be easy. "Conducted in the same apparatus" means, for example, a method of conducting the step C1 and then conducting the step D1 without taking the composition A out from the apparatus, or a method of continuously supplying the crude product A and the radical source into the apparatus in which the step D1 is to be conducted to conduct the step C1 and the step D1 simultaneously and continuously recovering the compound after the step D1. As a more specific example of this method, a method of conducting distillation while the radical source is supplied to the distillation column may, for example, be mentioned.

Another step may be conducted between the respective steps of the step A, the step C1 and the step D1 or after the step D1 in the first embodiment. For example, by conducting a step such as washing with water, separation by extraction or distillation between the step A and the step C1, a higher purity perfluorinated compound can be obtained more efficiently.

<Application>

In a case where the perfluoroallyl ether compound obtained by the method for producing a perfluorinated compound according to the first embodiment has a fluorosulfonyl group ($-SO_2F$), the perfluoroallyl ether compound is used suitably as a monomer component for production of a sulfonic acid group-containing fluorinated polymer.

The sulfonic acid group-containing fluorinated polymer is used suitably for production of an electrolyte membrane.

[Second embodiment]

The method for producing a perfluorinated compound in the second embodiment has the step B and the step C2.

<Step B>

The step B is a step of subjecting a chlorofluorocarbon compound to dechlorination reaction to obtain a crude product B containing a perfluorinated α-olefin compound.

A chlorofluorocarbon compound preferably has $-SO_2F$, whereby a perfluorinated α-olefin compound suitable for production of a polymer for an electrolyte membrane can be obtained, and is particularly preferably a compound represented by the following formula (S3) (hereinafter sometimes referred to as "compound (S3)").

$$ClCF_2\text{-}CFCl\text{-}CF_2\text{-}CF_2\text{-}Q^{13}\text{-}SO_2F \qquad (S3)$$

In the formula (S3), $Q^{13}$ is a single bond, a perfluoroalkylene group which may have $-SO_2F$, or a bivalent group having $-CF_2-$ in a perfluoroalkylene group which may have $-SO_2F$ replaced with an etheric oxygen atom.

The perfluoroalkylene group may be linear or branched.

The number of carbon atoms in the perfluoroalkylene group is, with a view to suppressing a decrease of the ion exchange capacity of the polymer, preferably from 1 to 12, more preferably from 1 to 6, particularly preferably from 1 to 4.

In a case where the perfluoroalkylene group has $-SO_2F$, the number of $-SO_2F$ may be one or more. $-SO_2F$ may be bonded to any carbon atom, but in a case where the perfluoroalkylene group is branched, it is bonded preferably to the terminal carbon atom.

The number of the etheric oxygen atom in the bivalent group may be one or more, and is preferably two or less. The etheric oxygen atom is preferably located in a carbon-carbon bond of the perfluoroalkylene group.

The step B is carried out preferably in the presence of an organic solvent. As specific examples of the organic solvent, an ether solvent such as dioxane, an alcohol organic solvent such as methanol, ethanol, isopropanol or n-butanol, a fatty acid organic solvent such as acetic acid, and an amide organic solvent such as N,N-dimethylformamide or N,N-dimethylacetamide may be mentioned.

In the step B, a metal such as zinc may further be used.

The reaction conditions in the step B are not particularly limited and for example, the reaction conditions as described in JP-A-2002-528433 may be employed.

(Crude product B)

The crude product B obtained by the step B contains the perfluorinated α-olefin compound which is a desired product and is one type of the perfluorinated compound, and further includes impurities other than the perfluorinated compound.

The impurities in the crude product B may, for example, be various compounds formed by side reactions and the organic solvent used in the step B.

The amount of the perfluorinated α-olefin compound contained in the crude product B is, to the total mass of the crude product B, preferably 30 mass% or more, more preferably 50 mass% or more, further preferably 85 mass% or more, particularly preferably 95 mass% or more.

The amount of the perfluorinated α-olefin compound contained in the crude product B is preferably large in view of the production efficiency, however, even if it is small, a perfluorinated α-olefin compound with a higher purity can be obtained e.g. by the after-described purification. Thus, a case where the amount of the perfluorinated α-olefin compound contained in the crude product B is small is also acceptable.

(Perfluorinated α-olefin compound)

The perfluorinated α-olefin compound preferably has a fluorosulfonyl group, whereby it is suitable for production of a polymer for an electrolyte membrane, and is particularly preferably a compound represented by the following formula (F2) (hereinafter sometimes referred to as "monomer (F2)").

$$CF_2{=}CF\text{-}CF_2\text{-}CF_2\text{-}Q^{13}\text{-}SO_2F \qquad (F2)$$

Definition of $Q^{13}$ in the formula (F2) is the same as that of $Q^{13}$ in the formula (S3).

The monomer (F2) is, in view of easy production of a polymer and high industrial applicability, preferably a compound represented by the following formula (F2-1) (hereinafter sometimes referred to as "monomer (F2-1)"), particularly preferably a compound represented by the following formula (F2-11) (hereinafter sometimes referred to as "monomer (F2-11)").

$$CF_2{=}CF\text{-}(CF_2CF_2)_{x2}\text{-}SO_2F \qquad (F2\text{-}1)$$

$$CF_2{=}CF\text{-}CF_2CF_2\text{-}SO_2F \qquad (F2\text{-}11)$$

In the formula (F2-1), x2 is an integer of from 1 to 6, preferably an integer of from 1 to 3, particularly preferably an integer of from 1 to 2.

<Step C2>

The step C2 is a step of mixing the crude product B with a radical source to bring radicals generated by the radical source and the crude product B into contact with each other to obtain a crude product C2 containing the perfluorinated α-olefin compound.

The crude product C2 obtained by the step C2 is considered to include, in addition to the desired product perfluorinated α-olefin compound, impurities originally included in the crude product B and impurities generated by decomposition or denaturation of the originally included impurities by the action of radicals. It is estimated that a higher molecular weight polymer is obtained by polymerizing the perfluorinated α-olefin compound since the impurities included in the crude product B become inactive to radicals by decomposition or denaturation by the action of radicals.

Specific examples and preferred embodiment of the radical source in the step C2 are as defined for the specific examples and the preferred embodiment of the radical source in the step C1.

The amount of the radical source added is, in view of more excellent effects of the present invention, preferably from 0.01 to 2 parts by mass, more preferably from 0.01 to 1 parts by mass, particularly preferably from 0.03 to 0.3 parts by mass, per 100 parts by mass of the crude product B.

The step C2 is conducted preferably under heating conditions. Preferred embodiments of the heating temperature and the heating time in the step C2 are as defined for the preferred embodiments of the heating temperature and the heating time in the step C1.

Mixing of the crude product B with the radical source is conducted preferably under conditions where substantially no polymerizable compound other than the polymerizable compound contained in the crude product B (hereinafter sometimes referred to as "other polymerizable compound B") is present, whereby consumption of radicals by other polymerizable compound B can be suppressed, and the reaction of radicals with impurities in the crude product B will be conducted more favorably.

Definition of the polymerizable compound and specific examples of the polymerizable group are as described above.

The polymerizable compound contained in the crude product B may, for example, be the perfluorinated α-olefin compound, and a compound having a polymerizable group in the impurities. The compound having a polymerizable

group in the impurities may, for example, be by-products formed by the step B.

Specific examples of the other polymerizable compound B are as defined for the specific examples of the other polymerizable compound.

In the step C2 "conditions where substantially no other polymerizable compound B is present", means that the content of the other polymerizable compound B is 0.1 parts by mass or less per 100 parts by mass of the crude product B used in the step C2.

The amount of the perfluorinated α-olefin compound contained in the crude product C2 is, to the total mass of the crude product C2, preferably 30 mass% or more, more preferably 50 mass% or more, further preferably 85 mass% or more, particularly preferably 95 mass% or more.

The amount of the perfluorinated α-olefin compound contained in the crude product C2 is preferably large in view of production efficiency, however, even if it is small, a perfluorinated α-olefin compound with a higher purity can be obtained e.g. by the after-described purification. Thus, a case where the amount of the perfluorinated α-olefin compound contained in the crude product C2 is small is also acceptable.

As described for the step C1, in order to sufficiently decompose or denature the impurities, it is preferred to conduct the step C2 under conditions where the perfluorinated α-olefin compound is decomposed to a certain extent. Specifically, the contact conditions are preferably such that the denaturation ratio D is a certain value or less.

The denaturation ratio D calculated by the following formula (M) is preferably as low as possible, whereby the yield of the perfluorinated compound increases. Specifically, the denaturation ratio D is preferably 20 % or less, more preferably 15 % or less, particularly preferably 5 % or less.

The lower limit of the denaturation ratio D is preferably 0%, however, in order that the impurities are securely decomposed or denatured, it is preferably 0.5%, more preferably 1.0%.

$$\text{Denaturation ratio D [\%]}=100\times(X-Y)/X \quad (M)$$

In the formula (M), X is the proportion (%) of the peak area of the perfluorinated α-olefin compound, to the total area of all peaks detected, in gas chromatography of the crude product B obtained in the step B.

In the formula (M), Y is the proportion (%) of the peak area of the perfluorinated α-olefin compound, to the total area of all peaks detected, in gas chromatography of the crude product C2 obtained in the step C2.

The denaturation ratio D can easily be adjusted to be the above value by adjusting the amount of the radical source added.

The crude product C2 containing the perfluorinated α-olefin compound thus obtained is preferably subjected to the after-described step D2, or it may be used as it is for the polymerization reaction. In such a case, it is preferred that the influence of the impurities included in the crude product C2 over the polymerization is small, and for example, it is preferred to use a perfluoroperoxide as the radical source, or to use the same solvent for the solvent used in the step B and the solvent for polymerization of the perfluorinated α-olefin compound.

<Step D2>

The method for producing a perfluorinated compound according to the second embodiment preferably has, after the step C2 in which the crude product C2 is obtained, step D2 to purify the crude product C2, whereby a perfluorinated compound, the perfluorinated α-olefin compound in the second embodiment, which has a high purity and is thereby used suitably for polymerization, can be obtained.

Specific examples and preferred embodiment of the purification method in the step D2 are as defined for the specific examples and the preferred embodiment of the purification method in the step D1.

The content of the perfluorinated α-olefin compound contained in the purified product obtained by the step D2 is, to the total mass of the purified product, preferably from 95 to 100 mass%., particularly preferably 99 to 100 mass%.

It is preferred that the step C2 and the step D2 are conducted in the same apparatus, whereby production of the perfluorinated compound will be easy.

Another step may be conducted between the respective steps of the step B, the step C2 and the step D2 or after the step D2 in the second embodiment. Specific examples of another step are as defined in the first embodiment.

<Application>

In a case where the perfluorinated α-olefin compound obtained by the method for producing a perfluorinated compound according to the second embodiment has a fluorosulfonyl group ($-SO_2F$), the perfluorinated α-olefin compound is used suitably as a monomer component for production of a sulfonic acid group-containing fluorinated polymer.

The sulfonic acid group-containing fluorinated polymer is used suitably for production of an electrolyte membrane.

[Method for producing fluorinated polymer]

The method for producing a fluorinated polymer of the present invention has a polymerization step of polymerizing the perfluorinated compound obtained by the above method (that is, the perfluoroallyl ether compound or the perfluorinated $\alpha$-olefin compound) at 100°C or more.

According to the method for producing a fluorinated polymer of the present invention, which uses the perfluorinated compound obtained by the above method, a high molecular weight fluorinated polymer can be obtained.

As a polymerization method, bulk polymerization, solution polymerization, suspension polymerization and emulsion polymerization may, for example, be mentioned.

The polymerization step is conducted preferably under conditions where radicals are generated. As a method of generating radicals, a method of irradiation with radial rays such as ultraviolet light, $\gamma$-ray or electron beams, or a method of adding a radical polymerization initiator may, for example, be mentioned.

Specific examples of the radical polymerization initiator are as defined for the radical polymerization initiator mentioned as the radical source. Particularly, it is preferred to use the polymerization initiator as described in Japanese Patent No. 5217708.

In the polymerization step, a monomer other than the perfluorinated compound (hereinafter sometimes referred to as "other monomer") may be used. Such other monomer may, for example, be tetrafluoroethylene (hereinafter sometimes referred to as "TFE"), chlorotrifluoroethylene, trifluoroethylene, vinylidene fluoride, vinyl fluoride, ethylene or propylene, and is particularly preferably TFE.

The polymerization temperature is 100°C or more, preferably 150°C or more, particularly preferably 160°C or more. When the polymerization temperature is 150°C or more, the fluorinated polymer production efficiency will be more excellent.

The polymerization temperature is preferably 190°C or less, particularly preferably 180°C or less. When the polymerization temperature is 190°C or less, the radical polymerization initiator decomposition rate will not be too high, and the polymerization will easily be controlled.

As the polymerization conditions other than the polymerization temperature, known conditions may properly be employed.

In a case where the fluorinated polymer obtained by the present production method has a fluorosulfonyl group ($-SO_2F$), it is suitably used as a precursor polymer for production of a sulfonic acid group-containing fluorinated polymer.

The Q value of the fluorinated polymer obtained by the present production method is preferably from 0.2 to 60.0 $mm^3$/sec, more preferably from 0.5 to 55 $mm^3$/sec, further preferably from 0.8 to 50 $mm^3$/sec, particularly preferably from 3.0 to 45 $mm^3$/sec.

A lower Q value of the fluorinated polymer means a higher molecular weight of the fluorinated polymer, and a higher Q value of the fluorinated polymer means a lower molecular weight of the fluorinated polymer. When the Q value of the fluorinated polymer is within the above range, the molecular weight of the fluorinated polymer is sufficiently high, and thus a polymer electrolyte membrane obtained by using the fluorinated polymer will be more excellent in hot water resistance. The Q value is measured by a method described in the following Examples.

EXAMPLES

Now, the present invention will be described in further detail with reference to Examples. Ex. 1-1 to 1-8, 2-1 to 2-8 and 3-1 to 3-8 are Examples of the present invention, and Ex. 1-9 to 1-13, 2-9 to 2-13 and 3-9 to 3-12 are Comparative Examples.

The crude product Y in Examples of the present invention will be referred to as "monomer F", and the crude product X in Comparative Examples will be referred to as "monomer F'". The fluorosulfonyl group-containing fluorinated polymer in Examples of the present invention will be referred to as "polymer F", and the fluorosulfonyl group-containing fluorinated polymer in Comparative Examples will be referred to as "polymer F'". Further, the sulfonic acid group-containing fluorinated polymer in Examples of the present invention will be referred to as "polymer H", and the sulfonic acid group-containing fluorinated polymer in Comparative Examples will be referred to as "polymer H'". However, it should be understood that the present invention is by no means restricted to such specific Examples.

The amount of components blended in the after-mentioned Tables are based on mass unless otherwise specified.

[Measurement of monomer purity]

The purity of the perfluorinated compound (represented as "monomer" in Tables) which is either the perfluoroallyl

ether compound or the perfluorinated α-olefin compound in the crude product was measured by gas chromatography (GC analysis) under the following conditions.

Apparatus: GC-2030 manufactured by Shimadzu Corporation
Column: DB-1 (length: 60m, inner diameter: 0.25 mm ID, thickness of liquid phase: 1.00 μm)
Oven temperature: 40°C (10 min)-10°C/min-240°C (25 min)
Inlet temperature: 250°C
Split ratio: 1/50
Control mode: pressure 100.0 kPa
Amount injected: 0.5 μL
Detector: FID
Detector temperature: 250°C

After the step A (or the step B) and before the step C1 (or the step C2), the crude product was subjected to GC analysis under the above conditions, and the proportion of the peak area of the perfluorinated compound which is either the perfluoroallyl ether compound or the perfluorinated α-olefin compound to the total area of all peaks detected was calculated and taken as the monomer purity X [%].
Further, after the step C1 (or the step C2) and the step D1 (or the step D2), the crude product was subjected to GC analysis under the above conditions, and the proportion of the peak area of the perfluorinated compound which is either the perfluoroallyl ether compound or the perfluorinated α-olefin compound to the total area of all peaks detected was calculated and taken as the monomer purity Y [%].
Further, after the step D1 (or the step D2), the purified product was subjected to GC analysis under the above conditions, and the proportion of the peak area of the perfluorinated compound which is either the perfluoroallyl ether compound or the perfluorinated α-olefin compound to the total area of all peaks detected was calculated and taken as the monomer purity Z [%].
From the obtained purities X and Y, in accordance with the following formula (M), the denaturation ratio D(%) was calculated.

$$\text{Denaturation ratio D [\%]} = 100\times(X-Y)/X \quad (M)$$

[Number of theoretical plate of distillation column]

Distillation of the crude product was conducted using a glass or stainless steel distillation column packed with random packing (HELIPACK No.1).
The number of theoretical plate of the distillation column was obtained by distilling a mixed solution containing two components having a known relative degree of volatility. Specifically, the degree of concentration was calculated from the rate of change of the compositional ratio of the two components in the distillate to the compositional ratio of the two components in the solution charged, and from the degree of concentration and the relative degree of volatility, the number of theoretical plate was calculated as follows.
Degree of concentration = compositional ratio of the two components in the distillate/compositional ratio of the two components in the solution charged
Number of theoretical plate = $Log_{10}$(degree pf concentration)/$Log_{10}$(relative degree of volatility)-1
In this specification, the number of theoretical plate was obtained by using a mixed solution of HCFC-225ca (chemical formula: $CF_3CF_2CHCl_2$) and HCFC-225cb (chemical formula: $CClF_2CF_2CHClF$) (relative degree of volatility=1.1423).

[Ion exchange capacity]

An acid-form sulfonic acid group-containing fluorinated polymer was vacuum dried at 120°C for 12 hours and dipped in a 0.85 mol/g sodium hydroxide solution (solvent: water/ethanol=10/90 (mass ratio)) to neutralize the ion exchange group. The sodium hydroxide solution after neutralization of the ion exchange group was subjected to back titration with 0.1mol/L hydrochloric acid to obtain the ion exchange capacity of the acid-form sulfonic acid group-containing fluorinated polymer.

[Proportion of constituents]

The proportions of the constituents such as TFE units and PSAE units in the fluorosulfonyl group-containing

fluorinated polymer were obtained from the results of $^{19}F$-NMR with respect to each polymer.

$^{19}F$-NMR was conducted under conditions of 282.7MHz, solvent: hexafluorobenzene, chemical shift standard: $CFCl_3$.

The proportions of the constituents in the sulfonic acid group-containing fluorinated polymer were the same as those in the fluorosulfonyl group-containing fluorinated polymer.

[Q value]

Using a flow tester (manufactured by Shimadzu Corporation, Capillary Rheometer Flowtester CFT-500D) equipped with a nozzle having an inner diameter of 1 mm and a length of 1 mm, polymer F or polymer F' filled in a cylinder having a cross sectional area of 1 $cm^2$ was extruded at 260°C under a load of 30 kg under a pressure of 2.94 MPa. The volume flow rate ($mm^3$/sec) of the polymer extruded at a time when the rate of extrusion of the polymer was stabilized, is taken as the Q value.

[Abbreviations]

For tetrafluoroethylene, the perfluoroallyl ether compound, the radical polymerization initiator and the organic solvent, the following abbreviations are used.

TFE: tetrafluoroethylene
PSAE: $CF_2=CFCF_2OCF_2CF_2SO_2F$
BSAE: $CF_2=CFCF_2OCF(-CF_2SO_2F)_2$
tBPO: $(CH_3)_3COOC(CH_3)_3$
PFtBPO: $(CF_3)_3COOC(CF_3)_3$
HFE-347pc-f: $CF_3CH_2OCF_2CF_2H$
HFC-52-13p: $CF_3(CF_2)_5H$
HCFC-141b: $CH_3CCl_2F$

[Production of perfluorinated compound]

<Ex. 1-1>

Crude product A-1 (monomer purity X: 97.00%) containing PSAE (perfluoroallyl ether compound) was synthesized by a synthesis route shown in the following scheme in accordance with the method described in Examples of US2005/0037265 (step A).

Scheme A

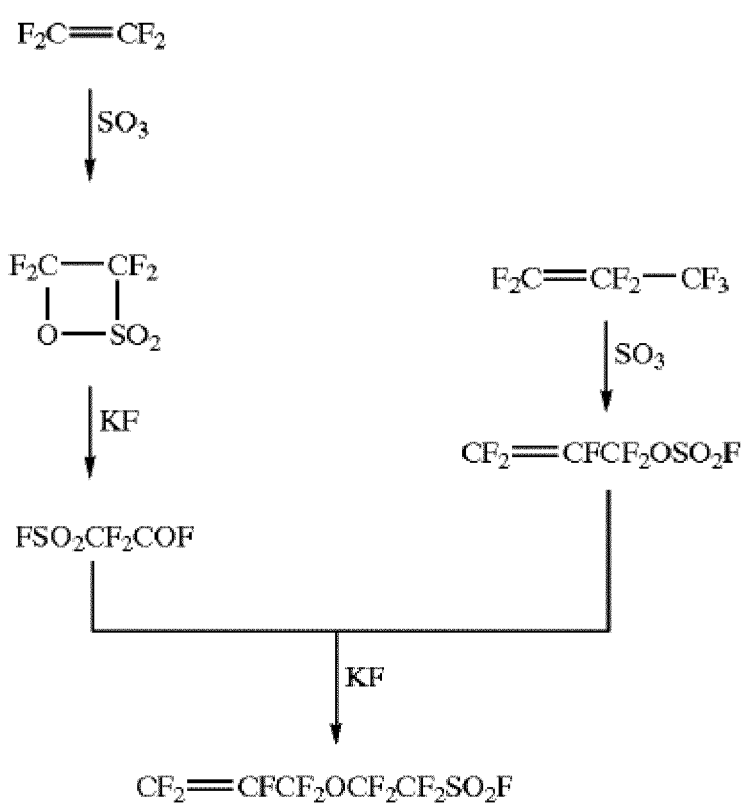


Into an autoclave (hereinafter abbreviated as A/C; internal capacity: 2500 mL, stainless steel), 2500 g of the crude product A-1 containing PSAE and 3.00 g (1200 ppm to the mass of the crude product A-1) of PFtBPO were put, and the autoclave was cooled with liquid nitrogen and deaerated. Nitrogen was introduced into a vapor phase, the autoclave was heated in an oil bath until the internal temperature reached 160°C, and the autoclave was heated for 3 hours while the internal temperature was kept at 160°C. The autoclave was cooled until the internal temperature reached 30°C or less, and the autoclave was purged of the gas to obtain crude product C1 -1 containing PSAE (step C1), which was taken as monomer F-1. Stirring during heating and cooling was conducted by a double helical ribbon blade, with a number of revolutions of 250 rpm. The results are shown in Table 1.

<Ex. 1-2>

Crude product A-2 (monomer purity X: 97.10%) containing PSAE (perfluoroallyl ether compound) was synthesized in the same manner as in Ex. 1-1 (step A).

Into an autoclave (hereinafter abbreviated as A/C; internal capacity: 1500 mL, stainless steel), 2029 g of the crude product A-2 containing PSAE and 0.7457 g (368 ppm to the mass of the crude product A-2) of tBPO were put, and the autoclave was cooled with liquid nitrogen and deaerated. Nitrogen was introduced into a vapor phase, the autoclave was heated in an oil bath until the internal temperature reached 160°C, and the autoclave was heated for 3 hours while the internal temperature was kept at 160°C. The autoclave was cooled until the internal temperature reached 30°C or less, and the autoclave was purged of the gas to obtain crude product C1-2 containing PSAE (step C1). Stirring during heating and cooling was conducted by an anchor blade, with a number of revolutions of 250 rpm.

Then, the crude product C1-2 was subjected to distillation to obtain a purified product containing PSAE (step D1). The distillation column was formed of glass, and the packing was HELIPACK No1.

The purified product containing PSAE was taken as monomer F-2. The results are shown in Table 1.

<Ex. 1-3>

Crude product A-3 (monomer purity X: 99.49%) containing PSAE (perfluoroallyl ether compound) was synthesized in the same manner as in Ex. 1-1 (step A).

In the same manner as in Ex. 1-2 except that the crude product A-3 thus obtained was used and that the

conditions were as identified in Table 1, crude product C1-3 and monomer F-3 (purified product containing PSAE) obtained by purifying the crude product C1-3 were obtained. The results are shown in Table 1.

<Ex. 1-4>

Crude product A-4 (monomer purity X: 99.82%) containing PSAE (perfluoroallyl ether compound) was synthesized in the same manner as in Ex. 1-1 (step A).

Then, in a distillation column (material: stainless steel, packing: HELIPACK No2, number of theoretical plate: 70, reflux ratio: 1) which was preliminarily deaerated and the interior of which was replaced with nitrogen, having an internal capacity of 40 L, 31.5 kg of the crude product A-4 containing PSAE and 25.2 g (800 ppm to the mass of the crude product A-4) of tBPO were put, and the distillation column was heated in an oil bas until the internal temperature reached 160°C and heated for 3 hours while the internal temperature was kept at 160°C. The distillation column was cooled until the internal temperature reached 30°C to obtain crude product C1-4 containing PSAE (step C1). The reflux ratio in this specification means the ratio of the amount of reflux to the amount of distillate (amount of reflux/amount of distillate).

Then, the crude product C1-4 was subjected to distillation as it was without being taken out from the distillation column to obtain purified product containing PSAE (step D1). The step C1 and the step D1 were carried out in the same reactor (distillation column).

The purified product containing PSAE was taken as monomer F-4. The results are shown in Table 1.

<Ex. 1-5>

In the same manner as in Ex. 1-2 except that the crude product A-2 obtained in Ex. 1-2 was used and that the conditions were as identified in Table 1, crude product C1-5 and monomer F-5 (purified product containing PSAE) obtained by purifying the crude product C1-5 were obtained. The results are shown in Table 1.

<Ex. 1-6>

Crude product A-6 (monomer purity X: 99.90%) containing PSAE (perfluoroallyl ether compound) was synthesized in the same manner as in Ex. 1-1 (step A).

Then, into an autoclave (hereinafter abbreviated as A/C; internal capacity: 1500 mL, stainless steel), 589.5 g of the crude product A-6 containing PSAE and 9.45 g (16000 ppm to the mass of the crude product A-6) of tBPO were put, and the autoclave was heated in an oil bath until the internal temperature reached 160°C, and the autoclave was heated for 3 hours while the internal temperature was kept at 160°C. The autoclave was cooled until the internal temperature reached 30°C to obtain a crude product C1-6 containing PSAE (step C1).

Then, the crude product C1-6 was subjected to distillation to obtain purified product containing PSAE (step D1). The distillation column was formed of glass, and the packing was HELIPACK No1.

The purified product containing PSAE was taken as monomer F-6. The results are shown in Table 1.

<Ex. 1-7>

Crude product A-7 (monomer purity X: 98.50%) containing BSAE (the perfluoroallyl ether compound) was synthesized by a synthesis route shown in the following scheme in accordance with the method described in Examples of WO2019/045063 (step A).

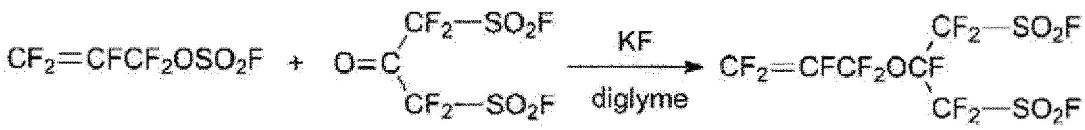


Then, into an autoclave (hereinafter abbreviated as A/C; internal capacity: 200 mL, stainless steel), 200 g of the crude product A-7 containing BSAE and 0.032 g (160 ppm to the mass of the crude product A-7) of tBPO were put, the autoclave was heated in an oil bath until the internal temperature reached 160°C, and the autoclave was heated for 3 hours while the internal temperature was kept at 160°C. The autoclave was cooled until the internal temperature reached 30°C to obtain crude product C1-7 containing BSAE (step C1).

Then, the crude product C1-7 was subjected to distillation to obtain purified product containing BSAE (step D1). The distillation column was formed of glass, and the packing was HELIPACK No1.

The purified product containing BSAE was taken as monomer F-7. The results are shown in Table 1.

<Ex. 1-8>

Crude product B-1 (monomer purity X: 99.44%) containing the perfluorinated α-olefin compound represented by the following formula (F2-1) was synthesized in accordance with the method described in Ex. 1 of JP-A-2002-528433 using a chlorofluorocarbon compound represented by the following formula (S3-1) (step A).

$CF_2$=CF-$CF_2$-$CF_2$-$SO_2$F (F2-1)

Cl$CF_2$-CFCl-$CF_2$-$CF_2$-$SO_2$F (S3-1)

Then, into an autoclave (hereinafter abbreviated as A/C; internal capacity: 200 mL, stainless steel), 200 g of the crude product B-1 containing the perfluorinated α-olefin compound represented by the formula (F2-1) and 0.08 g (400 ppm to the mass of the crude product B-1) of PFtBPO were put, and the autoclave was heated in an oil bath until the internal temperature reached 160°C, and the autoclave was heated for 3 hours while the internal temperature was kept at 160°C. The autoclave was cooled until the internal temperature reached 30°C to obtain crude product C2-1 containing the perfluorinated α-olefin compound represented by the formula (F2-1) (step C2).

Then, the crude product C2-1 was subjected to distillation to obtain purified product containing the perfluorinated α-olefin compound represented by the formula (F2-1) (step D2). The distillation column was formed of glass, and the packing was HELIPACK No1.

The purified product containing the perfluorinated α-olefin compound represented by the formula (F2-1) was taken as monomer F-8. The results are shown in Table 1.

<Ex. 1-9 to 1-11>

Monomers F'-1 to F'-3 (purified product containing PSAE) in Ex. 1-9 to 1-11 were obtained without conducting the step C1 in Ex. 1-1 and conducting only the step D1 (distillation) under the conditions as identified in Table 1. The results are shown in Table 1.

<Ex. 1-12>

Monomer F'-4 (purified product containing BSAE) in Ex. 1-7 was obtained without conducting the step C1 in Ex. 1-7 and conducting only the step D1 (distillation) under the conditions as identified in Table 1. The results are shown in Table 1.

<Ex. 1-13>

Monomer F'-5 (purified product containing the perfluorinated α-olefin compound represented by the formula (F2-1)) in Ex. 1-8 was obtained without conducting the step C2 in Ex. 1-8 and conducting only the step D2 (distillation) under the conditions as identified in Table 1. The results are shown in Table 1.

[Table 1]

| Table 1 | Ex. 1-1 | Ex. 1-2 | Ex. 1-3 | Ex. 1-4 | Ex. 1-5 | Ex. 1-6 | Ex. 1-7 | Ex. 1-8 |
|---|---|---|---|---|---|---|---|---|
| Monomer obtained | F-1 | F-2 | F-3 | F-4 | F-5 | F-6 | F-7 | F-8 |
| Monomer | *1 | *1 | *1 | *1 | *1 | *1 | *1 | *2 |
| Monomer purity X in crude product A or B [%] | 97.00 | 97.10 | 99.49 | 99.82 | 97.10 | 99.90 | 98.50 | 99.44 |
| Radical polymerization initiator | PFtBPO | tBPO | tBPO | tBPO | PFtBPO | tBPO | tBPO | PFtBPO |
| Capacity of reactor mL | 2500 | 1500 | 1500 | 40000 | 2500 | 1500 | 200 | 200 |
| Concentration of radical polymerization initiator added [mass ppm] | 1200 | 368 | 2250 | 800 | 1175 | 16000 | 160 | 400 |
| Concentration of radical polymerization initiator added [mol ppm] | 2.6 | 2.5 | 15.4 | 5.5 | 2.5 | 109.4 | 1.1 | 0.9 |
| Heating temperature [°C] | 160 | 160 | 160 | 160 | 160 | 160 | 160 | 160 |
| Heating time [h] | 3 | 3 | 6 | 3 | 3 | 3 | 3 | 3 |
| Monomer purity Y in crude product C1 or C2 [%] | 96.15 | 96.34 | 97.23 | 99.02 | 96.25 | 86.83 | 97.20 | 98.00 |
| Denaturation ratio D [%] | 0.9 | 0.8 | 2.3 | 0.8 | 0.9 | 13.1 | 1.3 | 1.4 |
| Purification method | Nil | Distillation | Distillation | Distillation | Distillation | Distillation | Distillation | Distillation |
| Number of theoretical plate of distillation column | - | 38.6 | 26.1 | 70 | 38.6 | 38.6 | 38.6 | 38.6 |
| Reflux ratio of distillation | - | 10 | 60 | 1 | 10 | 10 | 10 | 10 |
| *1: Perfluoroallyl ether, *2: Perfluorinated α-olefin | | | | | | | | |

[Table 1 (Continued)]

| Table 1 (Continued) | Ex. 1-9 | Ex. 1-10 | Ex. 1-11 | Ex. 1-12 | Ex. 1-13 |
|---|---|---|---|---|---|
| Monomer obtained | F'-1 | F'-2 | F'-3 | F'-4 | F'-5 |
| Monomer | *1 | *1 | *1 | *1 | *2 |
| Monomer purity X in crude product A or B [%] | 97.50 | 97.10 | 97.20 | 98.50 | 99.44 |
| Radical polymerization initiator | - | - | - | - | - |
| Capacity of reactor mL | | | | | |
| Concentration of radical polymerization initiator added [mass ppm] | - | - | - | - | - |
| Concentration of radical polymerization initiator added [mol ppm] | - | - | - | - | - |
| Heating temperature [°C] | - | - | - | - | - |
| Heating time [h] | - | - | - | - | - |
| Monomer purity Y in crude product C1 or C2 [%] | - | - | - | - | - |
| Denaturation ratio D [%] | - | - | - | - | - |
| Purification method | Distillation | Distillation | Distillation | Distillation | Distillation |
| Number of theoretical plate of distillation column | 120 × 6 times | 72.2 | 72.2 | 38.6 | 38.6 |
| Reflux ratio of distillation | 40 | 8 | 8 | 10 | 10 |
| *1: Perfluoroallyl ether, *2: Perfluorinated $\alpha$-olefin | | | | | |

[Production of polymer F and polymer F']

<Ex. 2-1>

An autoclave (internal capacity 2500 mL, stainless steel) was depressurized, and 2252.50 g of monomer F-1 (crude product containing PSAE) obtained in Ex. 1-1 was charged into the autoclave by suction. An operation of pressurization to 0.3 MPa (gauge pressure) by nitrogen gas and pressure relief to 0.05 MPa was repeated 5 times to remove dissolved oxygen. Nitrogen was introduced into a vapor phase, and the autoclave was heated in an oil bath until the internal temperature reached 160°C. On that occasion, the pressure was 1.101MPa (gauge pressure). Then, into the autoclave, 34.32 g of TFE was introduced. On that occasion, the pressure was 1.410 MPa (gauge pressure), and the TFE partial pressure at the polymerization temperature was 0.309 MPa.

0.72 g of an initiator solution having PFtBPO as an initiator dissolved in monomer F-1 at a concentration of 140 ppm was added to initiate copolymerization. On that occasion, the amount of PFtBPO added was 0.10 mg, and the concentration of PFtBPO in the autoclave was 0.045 ppm to the mass of monomer F-1 which had been charged in the autoclave before the start of copolymerization. TFE was continuously added while the pressure was kept at 1.410 MPa (gauge pressure) to continue the polymerization for 5 hours. During the polymerization, in order to keep the concentration of PFtBPO to the mass of the monomer F-1 which had been charged in the autoclave before the start of copolymerization at 0.045 ppm, the initiator solution was continuously added at a rate of 35.7 g/h (5.00 mg/h by the mass of PFtBPO) for 4 hours and 30 minutes. By the above operation, the total amount of monomer F-1 introduced into the autoclave was 2413.14 g, and the total amount of PFtBPO introduced to the autoclave was 22.5 mg. Accordingly, the ratio of the total amount of PFtBPO added to the total amount of monomer F-1 added was 1.9 ppm per hour of the polymerization time on average. Further, the amount of TFE later added was 22.20 g. Stirring during the copolymerization was conducted by a double helical ribbon blade, with a number of revolutions of 150 rpm.

The autoclave was cooled until the internal temperature reached 30°C or less, and the autoclave was purged of the gas. To the reaction liquid, 6033 g of HFE-347pc-f corresponding to 2.5 times (by mass) the amount of monomer F-1 were added to agglomerate the polymer, which was collected by filtration. Then, an operation of stirring the polymer

in HFE-347pc-f in the same amount to wash the polymer with HFE-347pc-f was repeated twice. The polymer was vacuum dried at 180°C to obtain 62.1 g of polymer F-1 which is a copolymer of TFE and PSAE. The Q value was 8.9 $mm^3$/sec. The results are shown in Table 2.

In Table 2, the productivity index (Rp) means the amount (g) of the polymer formed in 1 hour of the polymerization timer per 100 g of the total amount of monomer having a $SO_2F$ group charged before the copolymerization and during the copolymerization.

<Ex. 2-2>

Into an autoclave (hereinafter abbreviated as A/C; internal capacity 500mL, hastelloy) equipped with an air condenser, 525.0 g of monomer F-2 obtained in Ex. 1-2 (hereinafter referred to as purified product containing PSAE) was put, and the autoclave was cooled with liquid nitrogen and deaerated. Nitrogen was introduced into a vapor phase, and the autoclave was heated in an oil bath until the internal temperature reached 160°C. On that occasion, the pressure was 0.502 MPa (gauge pressure). Then, 10.40 g of TFE was introduced into the autoclave. On that occasion, the pressure was 0.892 MPa (gauge pressure), and the TFE partial pressure at the polymerization temperature was 0.390 MPa.

0.79 g of an initiator solution having tBPO as an initiator dissolved in monomer F-2 at a concentration of 667 ppm was added to initiate copolymerization. On that occasion, the amount of tBPO added was 0.53 mg, and the concentration of tBPO in the autoclave was 1.0 mass ppm to the mass of the monomer F-2 charged in the autoclave before the start of copolymerization. TFE was continuously added while the pressure was kept at 0.892 MPa (gauge pressure) to continue the polymerization for 5 hours. During the polymerization, 0.66 g (0.44 mg by the mass of tBPO) of the initiator solution was added 9 times every 30 minutes, to introduce totally 3.98 mg of tBPO into the autoclave. That is, tBPO was added so that the concentration of tBPO to the mass of monomer F-2 which had been charged in the autoclave before the start of copolymerization, would be 1.0 ppm immediately after the addition. By the above operation, the total amount of monomer F-2 introduced into the autoclave was 531.1 g, and the total amount of tBPO introduced to the autoclave was 4.51 mg. Accordingly, the ratio of the total amount of tBPO added to the total amount of monomer F-2 added was 1.7 ppm per hour of the polymerization time on average (in Tables, represented as "time average initiator ratio"). Further, the amount of TFE later added was 8.73 g. Stirring during the copolymerization was conducted by an anchor blade, with a number of revolutions of 250 rpm.

The autoclave was cooled until the internal temperature reached 30°C or less, and the autoclave was purged of the gas. To the reaction liquid, monomer F-2 and 1060 g of HFE-347pc-f corresponding to 2 times (by mass) the amount of monomer F-2 were added to agglomerate the polymer, which was collected by filtration. Then, an operation of stirring the polymer in HFE-347pc-f in the same amount to wash the polymer with HFE-347pc-f was repeated twice. The polymer was vacuum dried at 180°C to obtain 19.44 g of polymer F-2 which is a copolymer of TFE and PSAE. The Q value was 14.0 $mm^3$/sec. The results are shown in Table 2.

<Ex. 2-3>

Into an autoclave (hereinafter abbreviated as A/C; internal capacity 100mL, stainless steel) equipped with an air condenser, 80.02 g of monomer F-3 obtained in Ex. 1-3 (purified product containing PSAE) was put, and the autoclave was cooled with liquid nitrogen and deaerated. Nitrogen was introduced into a vapor phase, and the autoclave was heated in an oil bath until the internal temperature reached 160°C. On that occasion, the pressure was 0.460 MPa (gauge pressure). Then, 3.19 g of TFE was introduced into the autoclave. On that occasion, the pressure was 0.850 MPa (gauge pressure), and the TFE partial pressure at the polymerization temperature was 0.390 MPa.

0.32 g of an initiator solution having tBPO as an initiator dissolved in monomer F-3 at a concentration of 250 ppm was added to initiate copolymerization. On that occasion, the amount of tBPO added was 0.08 mg, and the concentration of tBPO in the autoclave was 1.0 mass ppm to the mass of monomer F-3 which had been charged in the autoclave before the start of copolymerization. TFE was continuously added while the pressure was kept at 0.850 MPa (gauge pressure) to continue the polymerization for 6 hours. During the polymerization, 0.27 g (0.07 mg by the mass of tBPO) of the initiator solution was added 11 times every 30 minutes, to introduce totally 0.77 mg of tBPO into the autoclave. That is, tBPO was added so that the concentration of tBPO to the mass of monomer F-3 which had been charged in the autoclave before the start of copolymerization, would be 1.0 ppm immediately after the addition. By the above operation, the total amount of monomer F-3 introduced into the autoclave was 83.31 g, and the total amount of tBPO introduced into the autoclave was 0.85 mg. Accordingly, the ratio of the total amount of tBPO added to the total amount of monomer F-3 added was 1.7 ppm per hour of the polymerization time on average (in Tables, represented as "time average initiator ratio"). Further, the amount of TFE later added was 1.72 g. Stirring during the copolymerization was conducted by a double helical ribbon blade, with a number of revolutions of 250 rpm.

The autoclave was cooled until the internal temperature reached 30°C or less, and the autoclave was purged of the gas. To the reaction liquid, monomer F-3 and 166 g of HFE-347pc-f corresponding to 2 times (by mass) the amount

of monomer F-3 were added to agglomerate the polymer, which was collected by filtration. Then, an operation of stirring the polymer in HFE-347pc-f in the same amount to wash the polymer with HFE-347pc-f was repeated twice. The polymer was vacuum dried at 180°C to obtain 2.8 g of polymer F-3 which is a copolymer of TFE and PSAE. The Q value was 7.9 $mm^3$/sec. The results are shown in Table 2.

<Ex. 2-4>

Polymer F-4 in Ex. 2-4 was obtained in the same manner as in Ex. 2-2 except that the conditions in Ex. 2-2 were changed as identified in Table 2. The results are shown in Table 2.

<Ex. 2-5>

Polymer F-5 in Ex. 2-5 was obtained in the same manner as in Ex. 2-1 except that the conditions in Ex. 2-1 were changed as identified in Table 2. The results are shown in Table 2.

<Ex. 2-6>

Polymer F-6 in Ex. 2-6 was obtained in the same manner as in Ex. 2-2 except that the conditions in Ex. 2-2 were changed as identified in Table 2. The results are shown in Table 2.

<Ex. 2-7>

Into an autoclave (hereinafter abbreviated as A/C; internal capacity 100mL, stainless steel) equipped with an air condenser, 69.99 g of monomer F-7 obtained in Ex. 1-7 (purified product containing PSAE) was put, and the autoclave was cooled with liquid nitrogen and deaerated. Nitrogen was introduced into a vapor phase, and the autoclave was heated in an oil bath until the internal temperature reached 160°C. On that occasion, the pressure was 0.300 MPa (gauge pressure). Then, 2.97 g of TFE was introduced into the autoclave. On that occasion, the pressure was 0.800 MPa (gauge pressure), and the TFE partial pressure at the polymerization temperature was 0.500 MPa.

0.18 g of an initiator solution having tBPO as an initiator dissolved in monomer F-7 at a concentration of 2000 ppm was added to initiate copolymerization. On that occasion, the amount of tBPO added was 0.35 mg, and the concentration of tBPO in the autoclave was 5.0 mass ppm to the mass of monomer F-7 which had been charged in the autoclave before the start of copolymerization. TFE was continuously added while the pressure was kept at 0.800 MPa (gauge pressure) to continue the polymerization for 8.5 hours. During the polymerization, 0.15 g (0.36 mg by the mass of tBPO) of the initiator solution was added 16 times every 30 minutes, to introduce totally 5.70 mg of tBPO into the autoclave. That is, tBPO was added so that the concentration of tBPO to the mass of monomer F-7 which had been charged in the autoclave before the start of copolymerization, would be 5.0 ppm immediately after the addition. By the above operation, the total amount of monomer F-7 introduced into the autoclave was 73.13 g, and the total amount of tBPO introduced into the autoclave was 6.05 mg. Accordingly, the ratio of the total amount of tBPO added to the total amount of monomer F-7 added was 9.7 mass ppm per hour of the polymerization time on average (in Tables, represented as "time average initiator ratio"). Further, the amount of TFE later added was 6.85 g. Stirring during the copolymerization was conducted by a double helical ribbon blade, with a number of revolutions of 180 rpm.

The autoclave was cooled until the internal temperature reached 30°C or less, and the autoclave was purged of the gas. The reaction liquid was diluted with HFC-52-13p, and HFE-347pc-f was added to agglomerate the polymer, which was collected by filtration. Then, an operation of stirring the polymer in HFC-52-13p to re-agglomerate the polymer with HFE-347pc-f was repeated twice. The polymer was vacuum dried at 120°C to obtain 13.92 g of polymer F-7 which is a copolymer of TFE and BSAE. The Q value was 27.2 $mm^3$/sec. The results are shown in Table 2.

<Ex. 2-8>

Polymer F-8 in Ex. 2-8 was obtained in the same manner as in Ex. 2-3 except that the conditions in Ex. 2-3 were changed as identified in Table 2, and except for the following. That is, in Ex 2-8, introduction of nitrogen into a vapor phase of the autoclave was not conducted. To the reaction liquid, monomer F-8 (purified product containing the perfluorinated $\alpha$-olefin compound represented by the formula (F2-1)) and 400 g of HCFC-141b corresponding to 4 times (by mass) the amount of monomer F-8 were added to agglomerate the polymer, which was collected by filtration. An operation of stirring the polymer in HCFC-141b in the same amount to wash the polymer with HCFC-141b was repeated twice. The results are shown in Table 2.

<Ex. 2-9 to 2-10>

Polymers F'-1 and F'-2 in Ex. 2-9 and 2-10 were obtained in the same manner as in Ex. 2-2 except that the conditions in Ex. 2-2 were changed as identified in Table 2. The results are shown in Table 2.

<Ex. 2-11>

Polymer F'-3 in Ex. 2-11 was obtained in the same manner as in Ex. 2-3 except that the conditions in Ex. 2-3 were changed as identified in Table 2. The results are shown in Table 2.

<Ex. 2-12>

Polymer F'-4 in Ex. 2-12 was obtained in the same manner as in Ex. 2-7 except that the conditions in Ex. 2-7 were changed as identified in Table 2. The results are shown in Table 2.

<Ex. 2-13>

Polymer F'-5 in Ex. 2-13 was obtained in the same manner as in Ex. 2-8 except that the conditions in Ex. 2-8 were changed as identified in Table 2. The results are shown in Table 2.

[Table 2-1]

| Table 2-1 | Ex. 2-1 | Ex. 2-2 | Ex. 2-3 | Ex. 2-4 | Ex. 2-5 | Ex. 2-6 | Ex. 2-7 | Ex. 2-8 |
|---|---|---|---|---|---|---|---|---|
| Monomer used | F-1 | F-2 | F-3 | F-4 | F-5 | F-6 | F-7 | F-8 |
| Capacity of reactor [mL] | 2500 | 500 | 100 | 500 | 2500 | 500 | 100 | 100 |
| Weight of monomer F or F' charged before start of copolymerization [g] | 2252.5 | 525 | 80.02 | 525 | 2257.2 | 525 | 69.99 | 70 |
| Weight of TFE charged before start of copolymerization [g] | 34.32 | 10.4 | 3.19 | 11.72 | 33.83 | 10.4 | 2.97 | 7.09 |
| Copolymerization temperature [°C] | 160 | 160 | 160 | 160 | 160 | 160 | 160 | 140 |
| TFE partial pressure [MPa] | 0.309 | 0.39 | 0.39 | 0.39 | 0.309 | 0.39 | 0.5 | 0.97 |
| Polymerization pressure [MPaG] | 1.41 | 0.892 | 0.850 | 0.897 | 1.409 | 0.892 | 0.8 | 0.97 |
| Initiator for copolymerization | PFtBPO | tBPO | tBPO | tBPO | PFtBPO | tBPO | tBPO | PFtBPO |
| Concentration of initiator solution added [ppm] | 140 | 667 | 250 | 667 | 140 | 667 | 2000 | 550 |
| Mass of initiator in A/C at start of copolymerization [mg] | 0.1 | 0.53 | 0.08 | 0.53 | 0.10 | 0.53 | 0.35 | 0.17 |
| Concentration of initiator to mass of monomer F or F' charged before start of copolymerization in A/C at start of copolymerization [ppm] | 0.045 | 1.0 | 1.0 | 1.0 | 0.045 | 1.0 | 5.0 | 2.4 |
| Method of later addition of initiator | *a | *b | *b | *b | *a | *b | *b | *b |
| Number of sequential addition | - | 9 | 11 | 9 | - | 9 | 16 | 63 |
| Amount of initiator per one addition [mg] | - | 0.44 | 0.07 | 0.44 | - | 0.44 | 0.36 | 0.19 |

(continued)

| Table 2-1 | Ex. 2-1 | Ex. 2-2 | Ex. 2-3 | Ex. 2-4 | Ex. 2-5 | Ex. 2-6 | Ex. 2-7 | Ex. 2-8 |
|---|---|---|---|---|---|---|---|---|
| Concentration of initiator to mass of monomer F or F' charged before start of | - | 1 | 1 | 1 | - | 1 | 5 | 2.4 |
| copolymerization in A/C immediately after sequential addition [ppm] | | | | | | | | |
| Rate of charge of initiator at continuous addition [mg/h] | 5 | - | - | - | 5 | - | - | - |
| Concentration of initiator to mass of monomer F or F' charged before start of copolymerization in A/C during continuous addition [ppm] | 0.045 | - | - | - | 0.045 | - | - | - |
| *a: Continuous addition, *b: Sequential addition every 30 minutes | | | | | | | | |

[Table 2-1 (Continued)]

| Table 2-1 (Continued) | Ex. 2-9 | Ex. 2-10 | Ex. 2-11 | Ex. 2-12 | Ex. 2-13 |
|---|---|---|---|---|---|
| Monomer used | F'-1 | F'-2 | F'-3 | F'-4 | F'-5 |
| Capacity of reactor [mL] | 500 | 500 | 100 | 100 | 100 |
| Weight of monomer F or F' charged before start of copolymerization [g] | 525 | 525 | 80.01 | 69.99 | 70 |
| Weight of TFE charged before start of copolymerization [g] | 10.67 | 11.84 | 2.51 | 2.92 | 7.05 |
| Copolymerization temperature [°C] | 160 | 160 | 160 | 160 | 140 |
| TFE partial pressure [MPa] | 0.445 | 0.39 | 0.39 | 0.47 | 0.97 |
| Polymerization pressure [MPaG] | 0.89 | 0.89 | 0.86 | 0.75 | 0.97 |
| Initiator for copolymerization | tBPO | tBPO | tBPO | tBPO | PFtBPO |
| Concentration of initiator solution added [ppm] | 667 | 667 | 250 | 2000 | 550 |
| Mass of initiator in A/C at start of copolymerization [mg] | 0.53 | 0.53 | 0.08 | 0.70 | 0.17 |
| Concentration of initiator to mass of monomer F or F' charged before start of copolymerization in A/C at start of copolymerization [ppm] | 1.0 | 1.0 | 1.0 | 10 | 2.4 |
| Method of later addition of initiator | *b | *b | *b | *b | *b |
| Number of sequential addition | 19 | 10 | 11 | 19 | 63 |
| Amount of initiator per one addition [mg] | 0.44 | 0.44 | 0.07 | 0.73 | 0.19 |
| Concentration of initiator to mass of monomer F or F' charged before start of copolymerization in A/C immediately after sequential addition [ppm] | 1 | 1 | 1 | 10 | 2.4 |
| Rate of charge of initiator at continuous addition [mg/h] | - | - | - | - | - |

(continued)

| Table 2-1 (Continued) | Ex. 2-9 | Ex. 2-10 | Ex. 2-11 | Ex. 2-12 | Ex. 2-13 |
|---|---|---|---|---|---|
| Concentration of initiator to mass of monomer F or F' charged before start of copolymerization in A/C during continuous addition [ppm] | - | - | - | - | - |
| *a: Continuous addition, *b: Sequential addition every 30 minutes | | | | | |

[Table 2-2]

| Table 2-2 | Ex. 2-1 | Ex. 2-2 | Ex. 2-3 | Ex. 2-4 | Ex. 2-5 | Ex. 2-6 | Ex. 2-7 | Ex. 2-8 |
|---|---|---|---|---|---|---|---|---|
| Copolymerization time [h] | 5 | 5 | 6 | 5 | 5 | 5 | 8.5 | 32 |
| Continuously added TFE [g] | 22.2 | 8.73 | 1.72 | 10.26 | 21.77 | 8.73 | 6.85 | 4.4 |
| Total amount of TFE added [g] | 56.52 | 19.13 | 4.91 | 21.98 | 55.6 | 19.13 | 9.82 | 11.49 |
| Total amount of monomer F or F' added [g] | 2413.14 | 531.1 | 83.31 | 531.1 | 2417.84 | 531.1 | 73.13 | 91.94 |
| Total amount of initiator added [mg] | 22.5 | 4.51 | 0.85 | 4.51 | 22.5 | 4.51 | 6.05 | 12.07 |
| Molar ratio of total amount of monomer F or F' added to total amount of TFE added | 12.9 | 8.4 | 5.1 | 7.3 | 13.2 | 8.4 | 1.7 | 3.0 |
| Lower limit of initiator concentration to mass of monomer F or F' charged before start of copolymerization in copolymerization [ppm] | 0.045 | 0.16 | 0.16 | 0.16 | 0.045 | 0.16 | 0.82 | 0.031 |
| Time average initiator ratio [ppm/h] | 1.9 | 1.7 | 1.7 | 1.7 | 1.9 | 1.7 | 9.7 | 4.1 |
| Polymer F, F' | F-1 | F-2 | F-3 | F-4 | F-5 | F-6 | F-7 | F-8 |
| Polymer yield [g] | 62.1 | 19.4 | 2.8 | 22.3 | 63.6 | 19.4 | 13.92 | 6.3 |
| Rp (productivity index) [g/(100 g·h)] | 0.51 | 0.73 | 0.56 | 0.84 | 0.53 | 0.73 | 2.24 | 0.22 |
| Monomer F or F' unit [mol%] | 32.3 | 29 | 26.6 | 29 | 32.5 | 29 | 19.6 | 14.6 |
| TFE unit [mol%] | 67.7 | 71 | 73.4 | 71 | 67.5 | 71 | 80.4 | 85.4 |
| Maximum value of initiator concentration to mass of monomer F or F' charged before start of copolymerization [ppm] | 0.045 | 1 | 1 | 1 | 0.045 | 1 | 5 | 2.4 |
| Q value [$mm^3$/sec] | 8.9 | 14.0 | 7.9 | 15.5 | 7.1 | 14.0 | 27.2 | 30.3 |

[Table 2-2 (Continued)]

| Table 2-2 (Continued) | Ex. 2-9 | Ex. 2-10 | Ex. 2-11 | Ex. 2-12 | Ex. 2-13 |
|---|---|---|---|---|---|
| Copolymerization time [h] | 10 | 5.5 | 6 | 10 | 32 |
| Continuously added TFE [g] | 20.5 | 9.24 | 0.66 | 3.58 | 4.5 |
| Total amount of TFE added [g] | 31.17 | 21.08 | 3.17 | 6.5 | 11.55 |
| Total amount of monomer F or F' added [g] | 537.5 | 532.43 | 83.34 | 76.15 | 92.1 |
| Total amount of initiator added [mg] | 8.87 | 4.95 | 0.83 | 14.58 | 12.14 |

(continued)

| Table 2-2 (Continued) | Ex. 2-9 | Ex. 2-10 | Ex. 2-11 | Ex. 2-12 | Ex. 2-13 |
|---|---|---|---|---|---|
| Molar ratio of total amount of monomer F or F' added to total amount of TFE added | 5.2 | 7.7 | 8.0 | 2.7 | 3.0 |
| Lower limit of initiator concentration to mass of monomer F or F' charged before start of copolymerization in copolymerization [ppm] | 0.16 | 0.16 | 0.16 | 1.6 | 0.031 |
| Time average initiator ratio [ppm/h] | 1.7 | 1.7 | 1.7 | 19.1 | 4.1 |
| Polymer F, F' | F'-1 | F'-2 | F'-3 | F'-4 | F'-5 |
| Polymer yield [g] | 42.7 | 23.5 | 0.6 | 6.63 | 6 |
| Rp (productivity index) [g/(100 g.h)] | 0.79 | 0.80 | 0.15 | 0.87 | 0.21 |
| Monomer F or F' unit [mol%] | 29.6 | 29.6 | 30.1 | 19.5 | 14.6 |
| TFE unit [mol%] | 70.4 | 70.4 | 69.9 | 80.5 | 85.4 |
| Maximum value of initiator concentration to mass of monomer F or F' charged before start of copolymerization [ppm] | 1 | 1 | 1 | 10 | 2.4 |
| Q value [$mm^3$/sec] | 15.5 | 1970 | Unmeasurable (oily) | 2100 | 2300 |

[Production of polymer H and polymer H']

<Ex. 3-1>

The polymer F-1 obtained as above was subjected to pressing at 260°C under 4 MPa (gauge pressure) to obtain a polymer F-1 film. The polymer F-1 film was dipped in an aqueous alkali solution (aqueous solution A: potassium hydroxide/water=20/80 (mass ratio)) at 80°C for 16 hours to hydrolyze and convert $-SO_2F$ in polymer F-1 into $-SO_3K$. Further, the polymer film was dipped in a 3 mol/L aqueous hydrochloric acid solution at 80°C for 30 minutes and then dipped in ultrapure water at 80°C for 30 minutes. The operation of dipping in an aqueous hydrochloric acid solution and dipping in ultrapure water was conducted totally five times to convert $-SO_3K$ in the polymer into $-SO_3H$. The polymer film was washed with ultrapure water repeatedly until the pH of water in which the polymer film was dipped became 7. The polymer film was sandwiched between filter papers and air dried to obtain a polymer H-1 film in Ex. 3-1. The results are shown in Table 3.

<Ex. 3-2 to 3-12>

Polymers H-2 to H-8 in Ex. 3-2 to 3-8, polymers H'-1 to H'-2 in Ex. 3-9 to Ex. 3-10, and polymers H'-4 to H'-5 in Ex. 3-11 to Ex. 3-12 were obtained in the same manner as in Ex. 3-1 except that polymer F-1 was changed to polymers F-2 to F-8, F'-1 to F'-2, and F'-4 to F'-5. The results are shown in Table 3.

[Table 3]

| Table 3-1 | Ex. 3-1 | Ex. 3-2 | Ex. 3-3 | Ex. 3-4 | Ex. 3-5 | Ex. 3-6 |
|---|---|---|---|---|---|---|
| Polymer used | F-1 | F-2 | F-3 | F-4 | F-5 | F-6 |
| Aqueous alkali solution used | Aqueous solution A | Aqueous solution A | Aqueous solution A | Aqueous solution A | Aqueous solution A | Aqueous solution A |
| Polymer obtained | H-1 | H-2 | H-3 | H-4 | H-5 | H-6 |
| Ion exchange capacity [meq/g dry resin] | 1.75 | 1.74 | 1.63 | 1.74 | 1.86 | 1.73 |

| Table 3-2 | Ex. 3-7 | Ex. 3-8 | Ex. 3-9 | Ex. 3-10 | Ex. 3-11 | Ex. 3-12 |
|---|---|---|---|---|---|---|
| Polymer used | F-7 | F-8 | F'-1 | F'-2 | F'-4 | F'-5 |
| Aqueous alkali solution used | Aqueous solution A | Aqueous solution A | Aqueous solution A | Aqueous solution A | Aqueous solution A | Aqueous solution A |
| Polymer obtained | H-7 | H-8 | H'-1 | H'-2 | H'-4 | H'-5 |
| Ion exchange capacity [meq/g dry resin] | 2.35 | 1.18 | 1.76 | 1.76 | 2.35 | 1.18 |

As shown in Tables 1 and 2, it was found that by conducting the step C1 or the step C2 at the time of monomer production, high molecular weight polymer F can be obtained even without conducting distillation at the time of monomer production, or even when distillation conditions at the time of monomer production are simplified (Ex. 1-1 to 1-8, Ex. 2-1 to 2-8).

On the other hand, it was found that to obtain high molecular weight polymer F'-1 by using monomer F'-1 (step C1 and step C2 not conducted), distillation conditions at the time of monomer production are complicated (Ex. 1-9, Ex. 2-9).

Further, it was further found that with monomers F'-2 to F'-5 obtained without conducting the step C1 and the step C2 and with simplified distillation conditions at the time of monomer production, high molecular weight polymer cannot be produced (Ex. 1-10 to 1-13, Ex. 2-10 to 2-13).

The entire disclosure of Japanese Patent Application No. 2021-112875 filed on July 7, 2021 including specification, claims and summary is incorporated herein by reference in its entirety.

## Claims

1. A method for producing a perfluorinated compound, which comprises step A of reacting a perfluoroalkoxide salt with a perfluoroallylating agent to obtain a crude product A containing a perfluoroallyl ether compound, or step B of subjecting a chlorofluorocarbon compound to dechlorination reaction to obtain a crude product B containing a perfluorinated $\alpha$-olefin compound, to obtain a crude product X which is either the crude product A or the crude product B, and
mixing the crude product X with a radical source to bring radicals generated by the radical source and the crude product X into contact with each other to obtain a crude product Y containing a perfluorinated compound which is either the perfluoroallyl ether compound or the perfluorinated $\alpha$-olefin compound.

2. The method for producing a perfluorinated compound according to Claim 1, wherein mixing of the crude product X with the radical source is conducted under conditions where substantially no polymerizable compound other than the polymerizable compound contained in the crude product X is present.

3. The method for producing a perfluorinated compound according to Claim 1 or 2, wherein the denaturation ratio D calculated by the following formula (M) is 15% or less:

$$\text{denaturation ratio D [\%]}=100\times(X-Y)/X \quad (M)$$

wherein X is the proportion (%) of the peak area of the perfluorinated compound to the total area of all peaks detected in gas chromatography of the crude product X, and Y is the proportion (%) of the peak area of the perfluorinated compound to the total area of all peaks detected in gas chromatography of the crude product Y.

4. The method for producing a perfluorinated compound according to any one of Claims 1 to 3, wherein the perfluoroalkoxide salt is a compound represented by the following formula (S1), and the perfluoroallylating agent is a compound represented by the following formula (S2):

$CF(-Q^{11})(-Q^{12})\text{-}O\text{-}M$ (S1)

$CF_2=CFCF_2\text{-}Z^S$ (S2)

in the formula (S1), $Q^{11}$ and $Q^{12}$ are each independently a perfluoroalkyl group which may have $-SO_2F$, a monovalent group having $-CF_2-$ in a perfluoroalkyl group which may have $-SO_2F$ replaced with an etheric oxygen atom, or a fluorine atom, and M is a monovalent metal element;
in the formula (S2), $Z^S$ is $-OS(O)_2R^{S1}$, a chlorine atom, a bromine atom or an iodine atom, and $R^{S1}$ is a fluorine atom or a perfluoroalkyl group.

**5.** The method for producing a perfluorinated compound according to Claim 4, wherein the perfluoroallyl ether compound obtained by reacting the compound represented by the formula (S1) with the compound represented by the formula (S2) is a compound represented by the following formula (F1):

$CF(-Q^{11})(-Q^{12})-O-CF_2CF=CF_2$ (F1)

in the formula (F1), definitions of $Q^{11}$ and $Q^{12}$ are respectively the same as those of $Q^{11}$ and $Q^{12}$ in the formula (S1).

**6.** The method for producing a perfluorinated compound according to Claim 4, wherein the compound represented by the formula (S1) is a compound represented by the following formula (S1-1) or a compound represented by the following formula (S1-2):

$FO_2S-Q^{21}-CF_2-O-M$ (S1-1)

$CF(-Q^{22}-SO_2F)(-Q^{23}-SO_2F)-O-M$ (S1-2)

in the formula (S1-1), $Q^{21}$ is a perfluoroalkylene group, or a bivalent group having $-CF_2-$ in a perfluoroalkylene group replaced with an etheric oxygen atom, and
in the formula (S1-2), $Q^{22}$ and $Q^{23}$ are each independently a perfluoroalkylene group, or a bivalent group having $-CF_2-$ in a perfluoroalkylene group replaced with an etheric oxygen atom.

**7.** The method for producing a perfluorinated compound according to Claim 6, wherein the perfluoroallyl ether compound obtained by reacting the compound represented by the formula (S1-1) with the compound represented by the formula (S2) is a compound represented by the following formula (F1-1), and the perfluoroallyl ether compound obtained by reacting the compound represented by the formula (S1-2) and the compound represented by the formula (S2) is a compound represented by the following formula (F1-2).

$FO_2S-Q^{21}-CF_2-O-CF_2CF=CF_2$ (F1-1)

$CF(-Q^{22}-SO_2F)(-Q^{23}-SO_2F)-O-CF_2CF=CF_2$ (F1-2)

in the formula (F1-1), definition of $Q^{21}$ is the same as that of $Q^{21}$ in the formula (S1-1); and
in the formula (F1-2), definitions of $Q^{22}$ and $Q^{23}$ are respectively the same as those of $Q^{22}$ and $Q^{23}$ in the formula (S1-2).

**8.** The method for producing a perfluorinated compound according to any one of Claims 1 to 3, wherein the chlorofluorocarbon compound is a compound represented by the following formula (S3):

$ClCF_2-CFCl-CF_2-CF_2-Q^{13}-SO_2F$ (S3)

in the formula (S3), $Q^{13}$ is a single bond, a perfluoroalkylene group which may have $-SO_2F$, or a bivalent group having $-CF_2-$ in a perfluoroalkylene group which may have $-SO_2F$ replaced with an etheric oxygen atom.

**9.** The method for producing a perfluorinated compound according to Claim 8, wherein the perfluorinated $\alpha$-olefin compound obtained by subjecting the chlorofluorocarbon compound to dechlorination reaction is a compound represented by the following formula (F2).

$CF_2=CF-CF_2-CF_2-Q^{13}-SO_2F$ (F2)

in the formula (F2), definition of $Q^{13}$ is the same as that of $Q^{13}$ in the formula (S3).

**10.** The method for producing a perfluorinated compound according to Claim 9, wherein the compound represented by

the formula (F2) is a compound represented by the following formula (F2-1).

$$CF_2=CF-(CF_2CF_2)_{X2}-SO_2F \qquad (F2-1)$$

in the formula (F2-1), x2 is an integer of from 1 to 6.

**11.** The method for producing a perfluorinated compound according to any one of Claims 1 to 10, wherein the crude product Y is further purified.

**12.** The method for producing a perfluorinated compound according to Claim 11, wherein mixing of the crude product X with the radical source, and purification of the crude product Y, are conducted in the same apparatus.

**13.** A method for producing a fluorinated polymer, which comprises polymerizing the perfluorinated compound obtained by the method for producing a perfluorinated compound as defined in any one of Claims 1 to 12 at 100°C or higher to obtain a fluorinated polymer.

## INTERNATIONAL SEARCH REPORT

International application No.
**PCT/JP2022/026340**

**A. CLASSIFICATION OF SUBJECT MATTER**

***C08F 216/14***(2006.01)i; ***C07C 309/82***(2006.01)i; ***C08F 214/18***(2006.01)i
FI: C08F216/14; C08F214/18; C07C309/82

According to International Patent Classification (IPC) or to both national classification and IPC

**B. FIELDS SEARCHED**

Minimum documentation searched (classification system followed by classification symbols)

C08F216/14; C07C309/82; C08F214/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | US 2005/0037265 A1 (ASAHI GLASS CO., LTD.) 17 February 2005 (2005-02-17) | 1-13 |
| A | JP 2002-528433 A (E.I. DU PONT DE NEMOURS AND CO.) 03 September 2002 (2002-09-03) | 1-13 |
| A | JP 46-23245 B1 (E. I. DU PONT DE NEMOURS AND CO.) 02 July 1971 (1971-07-02) | 1-13 |
| A | JP 2013-126760 A (DAIKIN INDUSTRIES, LTD.) 27 June 2013 (2013-06-27) | 1-13 |
| A | JP 2004-175855 A (UNIMATEC CO., LTD.) 24 June 2004 (2004-06-24) | 1-13 |
| A | WO 2019/045063 A1 (AGC INC.) 07 March 2019 (2019-03-07) | 1-13 |
| A | JP 2010-18674 A (ASAHI GLASS CO., LTD.) 28 January 2010 (2010-01-28) | 1-13 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/026340**

| Patent document cited in search report | Publication date (day/month/year) | Patent family member(s) | Publication date (day/month/year) |
|---|---|---|---|
| US 2005/0037265 A1 | 17 February 2005 | (Family: none) | |
| JP 2002-528433 A | 03 September 2002 | WO 2000/024709 A2<br>EP 1124796 A1<br>KR 10-2001-0080900 A | |
| JP 46-23245 B1 | 02 July 1971 | GB 1210794 A<br>DE 1901872 A<br>FR 1600355 A<br>NL 6900832 A | |
| JP 2013-126760 A | 27 June 2013 | US 2010/0314153 A1<br>WO 2008/047906 A1 | |
| JP 2004-175855 A | 24 June 2004 | (Family: none) | |
| WO 2019/045063 A1 | 07 March 2019 | US 2020/0190025 A1<br>EP 3677570 A1<br>CN 111065624 A<br>KR 10-2020-0047516 A | |
| JP 2010-18674 A | 28 January 2010 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010018674 A
- JP 2002528433 A
- JP 5217708 B
- US 20050037265 A
- WO 2019045063 A
- JP 2021112875 A